# EUROPEAN PATENT SPECIFICATION

(11) **EP 1 845 088 B1**
(45) Date of publication and mention of the grant of the patent: **22.07.2009**
(21) Application number: 07105903.4
(22) Date of filing: 10.04.2007
(51) Int. Cl.: C07D 215/22, A61K 31/47

(54) **An improved process for the preparation of aripipirazole**
Verbesserter Herstellungsprozess für Aripipirazol
Procédé amélioré pour la préparation d'aripipirazole

(30) Priority: 10.04.2006 IN DE09772006
(43) Date of publication of application: 17.10.2007
(73) Proprietor: Ranbaxy Laboratories Limited, Gurgaon - 122001, Haryana (IN)
(72) Inventor: Tewari, Neera, Gurgaon, Haryana 122001 (IN); Nizar, Hashim, 689645, Pathanamthitta Kerala (IN); Rai, Bishwa Prakash, 276306, Uttar Pradesh (IN)
(74) Representative: Cronin, Brian Harold John

(56) References cited:
- US-A- 5 006 528
- US-A1- 2004 192 915
- US-A1- 2005 215 791
- US-A1- 2006 079 689

## Description

### Field Of Invention

The present invention provides a process for the preparation of aripiprazole.

### Background Of The Invention

Aripiprazole is chemically 7-{4-[4-(2,3-dichlorophenyl)-1-piperazinyl]butoxy-3,4-dihydro-2(H)-quinolinone and is represented by Formula I.

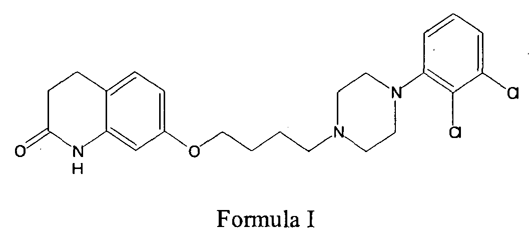

It is known from U.S. Patent 5,006,528 and is useful as an atypical antipsychotic agent for treating Schizophrenia. Several processes have been reported for the preparation of aripiprazole such as those described in U.S. Patent 5,006,528, U.S. Patent Application 2004/0192915 and U.S. Patent Application 2005/0215791.

U.S. Patent 5,006,528 describes the preparation of aripiprazole comprising reacting a carbostyril compound with dichlorophenyl piperazine in acetonitrile in the presence of triethylamine and sodium iodide.

U.S. Patent Application 2004/0192915 discloses the preparation of aripiprazole comprising reacting a carbostyril compound with dichlorophenyl piperazine in water in the presence of an inorganic base in a specific amount.

U.S. Patent Application 2005/0215791 describes the preparation of aripiprazole comprising reacting a carbostyril compound with dichlorophenyl piperazine hydrochloride in organic solvent in the presence of inorganic base and a phase transfer catalyst such as dodecyl sulfate sodium salt, tetrabutylammonium bromide or hexadecyl trimethylammonium bromide.

### Summary of the Invention

The present invention provides a process for the preparation of aripiprazole comprising condensing carbostyril compound with dichlorophenyl piperazine or its salts in water in the presence of an organic base.

### Detailed Description of the Invention

The carbostyril compound used as starting material in the present invention may be represented by Formula II 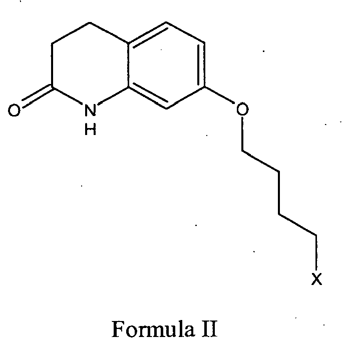 wherein X is a leaving group including a halogen atom, a lower alkanesulfonyloxy group, an arylsulfonyloxy group or an aralkylsulfonyloxy group.

When X is a halogen atom, it can be selected from the group consisting of fluorine, chorine, bromine and iodine. Preferably 7-(4-Bromobutoxy)-3,4-dihydrocarbostyril is used in some particular embodiments.

Examples of lower alkanesulfonyloxy group include methanesulfonyloxy group, ethanesulfonyloxy group, isopropanesulfonyloxy group, n-propanesulfonyloxy group, n-butanesulfonyloxy group, tert-butanesulfonyloxy group, n-pentanesulfonyloxy group or n-hexanesulfonyloxy group.

Examples of an arylsulfonyloxy group include phenylsulfonyloxy group, 4-methylphenylsulfonyloxy group, 2-methylphenylsulfonyloxy group, 4-nitrophenylsulfonyloxy group, 4-methoxyphenylsulfonyloxy group, 2-nitrophenylsulfonyloxy group, 3-nitrophenylsulfonyloxy group or 3-chlorophenylsulfonyloxy group.

Dichlorophenyl piperazine salts used as starting material in the present invention may be represented by the Formula III. 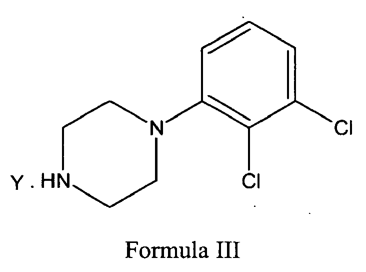 wherein Y is an organic or inorganic acid.

Examples of organic acid include oxalic acid, maleic acid, fumaric acid, tartaric, citric acid or benzoic acid. Examples of inorganic acid include hydrochloric acid, hydrobromic acid, sulfuric acid or phosphoric acid. 1-(2,3-dichlorophenyl)piperazine hydrochloride is used as the preferred starting material in some particular embodiments.

The carbostyril compounds and dichlorophenyl piperazine or its salts used as starting material in the present invention are known compounds and may be obtained from the methods known in the literature including those as described in U.S. Patent 5,002,528 and U.S. Patent Application 2005/0215585, which are herein incorporated by reference.

Examples of organic base used in the condensation reaction may include trimethylamine, triethylamine, tributylamine, triisopropylamine, diisopropylethylamine, tetramethyl guanidine, DBU (1,8-diazabicyclo- [5.4.0]-undec-7-ene), DBN (1,5-diazabicyclo-[4.3.0]-non-5-ene), 4-dimethylamino pyridine or mixtures thereof

The condensation reaction may be carried out at a temperature ranging from about 20°C to about 200°C. Preferably, the condensation reaction may be carried out at about 40 to 100°C. The reaction may be carried out for about 1 to 10 hours.

The product obtained from the reaction mixture may be isolated by conventional methods. Isolation may be accomplished by concentration, crystallization, precipitation, cooling, filtration, centrifugation or a combination thereof

If needed, the product obtained may be recrystallized from a suitable solvent or mixture of solvents. Suitable solvent include lower alkyl alcohols having 1-5 carbons, such as methanol, ethanol, isopropanol and butanol; ketones such as acetone and methyl isobutyl ketone; nitriles such as acetonitrile; chlorinated hydrocarbons such as methylene chloride, ethylene dichloride and carbon tetrachloride; esters such as ethyl acetate and isopropyl acetate; polar aprotic solvents such as dimethyl sulfoxide and dimethyl formamide; cyclic ethers such as dioxane and tetrahydrofuran; alkyl ethers such as diethyl ether, diisopropyl ether and dimethoxyethane and mixtures thereof.
In the following section preferred embodiments are described by way of examples to illustrate the process. Several variants of these examples would be evident to persons ordinarily skilled in the art.

### Examples

### Example-1

### Preparation of 7-{4-[4-(2,3-dichlorophenyl)-1-piperazinyl]butoxy-3,4-dihydro-2(H)-quinolinone (Aripiprazole):

7-(4-Bromobutoxy)-3,4-dihydrocarbostyril (50 g), 1-(2,3-dichlorophenyl)piperazine hydrochloride (50 g) and triethylamine (34 g) were suspended in water (500 ml). The above mixture was warmed to 40 to 50°C and stirred for 4 hours at that temperature. The temperature was further raised to 80 to 90°C and the stirring continued for 2 hours at 80 to 90°C. The resulting slurry was then cooled to between 20 and 25°C, filtered and washed with water (500 ml).

The wet cake was suspended in denatured spirit (1500 ml) and heated to reflux temperature. The solution was filtered in hot. The filtrate was cooled to 0 to 5°C to obtain pure aripiprazole (71 g). HPLC Purity: 99.0 % Melting point: 138-140°C

### Example-2

### Preparation of 7-{4-[4-(2,3-dichlorophenyl)-1-piperazinyl]butoxy-3,4-dihydro-2(H)-quinolinone (Aripiprazole):

7-(4-Bromobutoxy)-3,4-dihydrocarbostyril (50 g), 1-(2,3-dichlorophenyl)piperazine hydrochloride (50 g) and triethylamine (34 g) were suspended in water (500 ml). The above suspension was warmed to 40 to 50°C and stirred for 4 hours at that temperature. The temperature was further raised to 80 to 90°C and the stirring continued for 2 hours at 80 to 90°C. The resulting slurry was then cooled to between 20 and 25°C, filtered and washed with water (500 ml). The product was dried at 80°C for 3 to 4 hours to obtain crude aripiprazole (74 g).

The material so obtained was dissolved in ethanol (1500 ml) and heated to reflux temperature. The solution was filtered in hot. The filtrate was cooled to 0 to 5°C to obtain pure aripiprazole (68 g). HPLC Purity: 99.0%. Melting point: 139-140°C

### Example-3

### Preparation of 7-{4-[4-(2,3-dichlorophenyl)-1-piperazinyl]butoxy-3,4-dihydro-2(H)-quinolinone (Aripiprazole):

7-(4-Bromobutoxy)-3,4-dihydrocarbostyril (50 g), 1-(2,3-dichlorophenyl)piperazine hydrochloride (50 g) and tetramethyl guanidine (39.7g) were suspended in water (500 ml). The above mixture was warmed to 40 to 50°C and stirred for 4 hours. The temperature was further raised to 80 to 90°C and the stirring continued for 2 hours at 80 to 90°C. The resulting slurry was then cooled to between 20 and 25°C, filtered and washed with water (500 ml). The product was dried at 80°C for 3 to 4 hours to obtain crude aripiprazole (75 g)

The above material was dissolved in ethanol (1500 ml) and heated to reflux temperature. The solution was filtered in hot. The filtrate was cooled to 0 to 5°C to obtain pure aripiprazole (62.5 g). HPLC Purity: 99.0%. Melting point: 139-140°C

## Claims

1. A process for the preparation of aripiprazole of Formula I 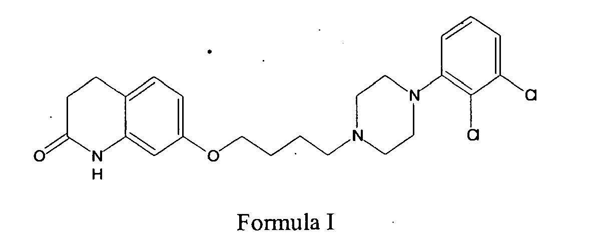 comprising condensing carbostyril compound of Formula II 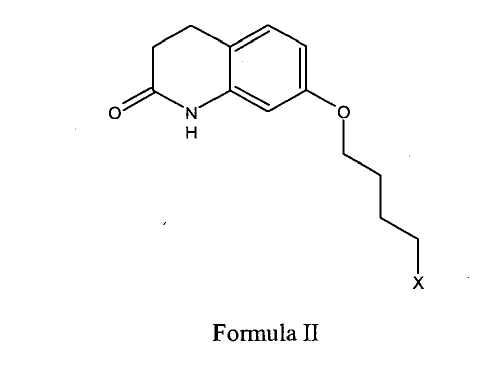 wherein X is a leaving group with dichlorophenyl piperazine or its salts of Formula III 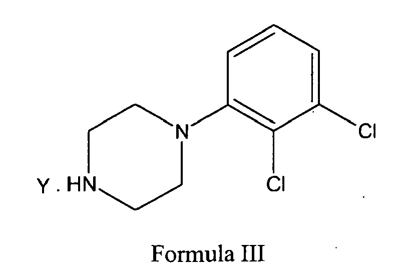 wherein Y is an organic or inorganic acid in water in the presence of an organic base.

2. The process according to claim 1, wherein the leaving group is selected from a halogen atom, a lower alkanesulfonyloxy group, an arylsulfonyloxy group or an aralkylsulfonyloxy group.

3. The process according to claim 2, wherein halogen is selected from fluorine, chlorine, bromine or iodine.

4. The process according to claim 2, wherein lower alkanesulfonyloxy group is selected from methanesulfonyloxy group, ethanesulfonyloxy group, isopropanesulfonyloxy group, n-propanesulfonyloxy group, n-butanesulfonyloxy group, tert-butanesulfonyloxy group, n-pentanesulfonyloxy group or n-hexanesulfonyloxy group.

5. The process according to claim 2, wherein arylsulfonyloxy group is selected from phenylsulfonyloxy group, 4-methylphenylsulfonyloxy group, 2-methylphenylsulfonyloxy group, 4-nitrophenylsulfonyloxy group, 4-methoxyphenylsulfonyloxy group, 2-nitrophenylsulfonyloxy group, 3-nitrophenylsulfonyloxy group or 3-chlorophenylsulfonyloxy group.

6. The process according to claim 1, wherein the organic acid is selected from the group comprising of oxalic acid, maleic acid, fumaric acid, tartaric, citric acid or benzoic acid.

7. The process according to claim 1, wherein inorganic acid is selected from the group comprising of hydrochloric acid, hydrobromic acid, sulfuric acid or phosphoric acid.

8. The process according to claim 1, wherein organic base is selected from of trimethylamine, triethylamine, tributylamine, triisopropylamine, diisopropylethylamine, tetramethyl guanidine, DBU (1,8-diazabicyclo- [5.4.0]-under-7-ene), DBN (1,5-diazabicyclo-[4.3.0]-non-5-ene), 4-dimethylamino pyridine or mixtures thereof.

9. The process according to claim 1, wherein condensation reaction is carried out at a temperature ranging from about 20°C to about 200° C.

10. The process according to claim 1, wherein condensation reaction is carried out for about 1 to 10 hours.

## Patentansprüche

1. Verfahren zur Herstellung von Aripiprazol nach Formel I 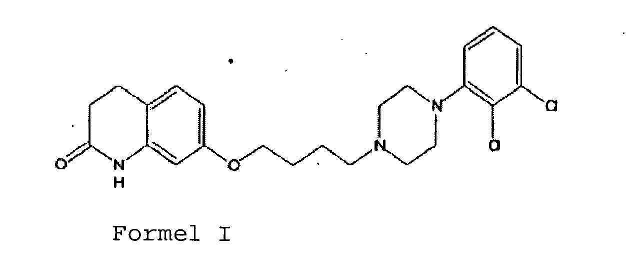 umfassend eine Kondensation der Carbostyril-Verbindung nach Formel II 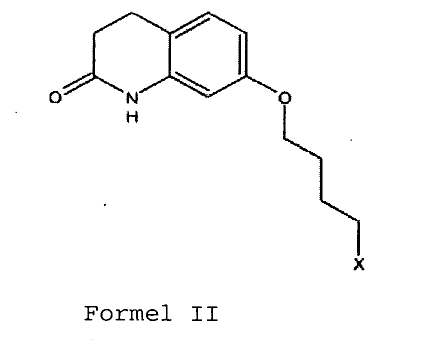 wobei X eine Abgangsgruppe mit Dichlorophenylpiperazin oder dessen Salzen nach Formel III ist 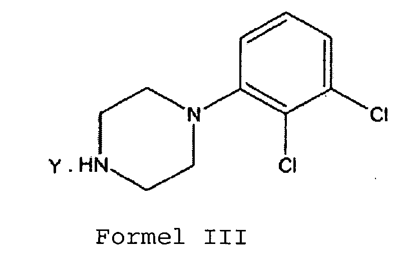 wobei Y eine organische oder anorganische Säure in Wasser bei Anwesenheit einer organischen Base ist.

2. Verfahren nach Anspruch 1, wobei die Abgangsgruppe ausgewählt ist aus einem Halogenatom, einer niedrigen Alkansulfonyloxygruppe, einer Arylsulfonyloxygruppe oder einer Aralkylsulfonyloxygruppe.

3. Verfahren nach Anspruch 2, wobei Halogen ausgewählt ist aus Fluor, Chlor, Brom oder Jod.

4. Verfahren nach Anspruch 2, wobei die niedrige Alkansulfonyloxygruppe ausgewählt ist aus Methansulfonyloxygruppe, Ethansulfonyloxygruppe, Isopropansulfonyloxygruppe, n-Propansulfonyloxygruppe, n-Butansulfonyloxygruppe, tert-Butansulfonyloxygruppe, n-Pentansulfonyloxygruppe oder n-Hexansulfonyloxygruppe.

5. Verfahren nach Anspruch 2, wobei die Arylsulfonyloxygruppe ausgewählt ist aus Phenylsulfonyloxygruppe, 4-Methylphenylsulfonyloxygruppe, 2-Methylphenylsulfonyloxygruppe, 4-Nitrophenylsulfonyloxygruppe, 4-Methoxyphenylsulfonyloxygruppe, 2-Nitrophenylsulfonyloxygruppe, 3-Nitrophenylsulfonyloxygruppe oder 3-Chlorophenylsulfonyloxygruppe.

6. Verfahren nach Anspruch 1, wobei die organische Säure ausgewählt ist aus der Gruppe enthaltend Oxalsäure, Maleinsäure, Fumarsäure, Weinsäure, Zitronensäure oder Benzoesäure.

7. Verfahren nach Anspruch 1, wobei die anorganische Säure ausgewählt ist aus der Gruppe enthaltend Salzsäure, Bromwasserstoffsäure, Schwefelsäure oder Phosphorsäure.

8. Verfahren nach Anspruch 1, wobei die organische Base ausgewählt ist aus Trimethylamin, Triethylamin, Tributylamin, Triisopropylamin, Diisopropylethylamin, Tetramethylguanidin, DBU(1,8-Diazabicyclo-[5.4.0]-undec-7-en), DBN(1,5-Diazabicyclo-[4.3.0]-non-5-en), 4-Dimethylaminopyridin oder Mischungen derselben.

9. Verfahren nach Anspruch 1, wobei die Kondensationsreaktion bei einer Temperatur im Bereich von ungefähr 20°C bis ungefähr 200°C durchgeführt wird.

10. Verfahren nach Anspruch 1, wobei die Kondensationsreaktion für einen Zeitraum von ungefähr 1 bis 10 Stunden durchgeführt wird.

## Revendications

1. Procédé pour la préparation d'aripiprazole de Formule I 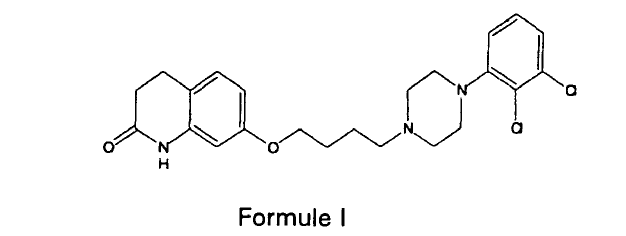 consistant à condenser un composé carbostyrile de Formule II 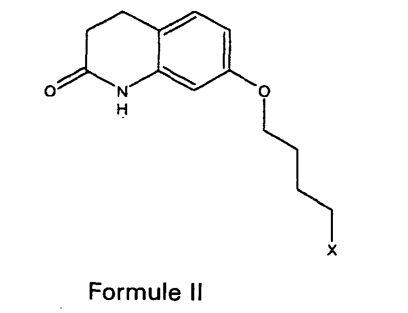 où X est un groupe partant avec de la dichlorophénylpipérazine ou ses sels de Formule III 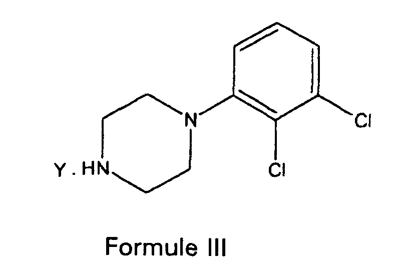 où Y est un acide organique ou inorganique dans de l'eau en présence d'une base organique.

2. Procédé selon la revendication 1, dans lequel le groupe partant est choisi parmi un atome d'halogène, un groupe alkanesulfonyloxy inférieur, un groupe arylsulfonyloxy ou un groupe aralkylsulfonyloxy.

3. Procédé selon la revendication 2, dans lequel l'halogène est choisi parmi le fluor, le chlore, le brome ou l'iode.

4. Procédé selon la revendication 2, dans lequel le groupe alkanesulfonyloxy inférieur est choisi parmi un groupe méthanesulfonyloxy, un groupe éthanesulfonyloxy, un groupe isopropanesulfonyloxy, un groupe n-propanesulfonyloxy, un groupe n-butanesulfonyloxy, un groupe tert-butanesulfonyloxy, un groupe n-pentanesulfonyloxy ou un groupe n-hexanesulfonyloxy.

5. Procédé selon la revendication 2, dans lequel le groupe arylsulfonyloxy est choisi parmi un groupe phénylsulfonyloxy, un groupe 4-méthylphénylsulfonyloxy, un groupe 2-méthylphénylsulfonyloxy, un groupe 4-nitrophénylsulfonyloxy, un groupe 4-méthoxyphénylsulfonyloxy, un groupe 2-nitrophénylsulfonyloxy, un groupe 3-nitrophénylsulfonyloxy ou un groupe 3-chlorophénylsulfonyloxy.

6. Procédé selon la revendication 1, dans lequel l'acide organique est choisi parmi le groupe constitué de l'acide oxalique, l'acide maléique, l'acide fumarique, l'acide tartrique, l'acide citrique ou l'acide benzoïque.

7. Procédé selon la revendication 1, dans lequel l'acide inorganique est choisi parmi le groupe constitué de l'acide chlorhydrique, l'acide bromhydrique, l'acide sulfurique ou l'acide phosphorique.

8. Procédé selon la revendication 1, dans lequel la base organique est choisie parmi la triméthylamine, la triéthylamine, la tributylamine, la triisopropylamine, la diisopropyléthylamine, la tétraméthylguanidine, le DBU (1,8-diazabicyclo-[5.4.0]-undéc-7-ène), le DBN (1,5-diazabicyclo-[4.3.0]-non-5-ène), la 4-diméthylaminopyridine ou des mélanges de ceux-ci.

9. Procédé selon la revendication 1, dans lequel la réaction de condensation est réalisée à une température allant d'environ 20°C à environ 200°C.

10. Procédé selon la revendication 1, dans lequel la réaction de condensation est réalisée pendant environ 1 à 10 heures.
